# EUROPEAN PATENT SPECIFICATION

(11) **EP 2 591 717 B1**
(45) Date of publication and mention of the grant of the patent: **31.08.2016**
(21) Application number: 11755439.4
(22) Date of filing: 08.07.2011
(51) Int. Cl.: A61B 5/01, A41B 11/00, A41D 13/12, A61B 5/0205, A61B 5/107, A61B 5/00, A61B 5/024, A61B 5/103, A61B 5/11

(54) **SOCK FOR INTEGRATED BIOMETRIC MONITORING**
SOCKE FÜR INTEGRIERTE BIOMETRISCHE ÜBERWACHUNG
CHAUSSETTE POUR SURVEILLANCE BIOMÉTRIQUE INTÉGRÉE

(30) Priority: 08.07.2010 PT 10519110
(43) Date of publication of application: 15.05.2013
(73) Proprietor: Fiorima S.A., 4700-155 Braga (PT)
(72) Inventor: Pinto Rodrigues, Paulo Jorge, P-4700 - 352 Braga (PT)
(74) Representative: Ferreira, Maria Silvina
(86) International application number: PCT/IB2011/053058
(87) International publication number: WO 2012/004774

(56) References cited:
- WO-A1-2009/023937
- DE-A1- 10 314 211
- US-A1- 2005 034 485
- US-A1- 2005 054 941
- US-A1- 2008 307 899
- US-B1- 6 216 545

## Description

### Technical field of the invention

The present invention relates to a sock with integrated biometric monitoring.

### Summary of the invention

The present invention is defined in the independent claim 1, which is partitioned relative to the document WO 2009/023937 A1.

A preferred embodiment comprises a data processing device (11) and a conductor connection band (8) through which binds to the data bus (7), and that includes wireless interface (12), in particularly to a mobile device (15).

A preferred embodiment comprises a data processing device (11) which includes one or more visual indicators, in particular LED (13), one or more buttons (14), in particular an on / off switch.

In a preferred embodiment the referred sensors comprise a pressure sensor (5) and a temperature sensor (1).

In a preferred embodiment the referred sensors comprise connections to the data bus (7) through conductive yarns in "zig-zag".

In a preferred embodiment one or more of the referred sensors comprisse connections to the data bus (7) through conductive yarns in "zig-zag".

In a preferred embodiment one or more of the referred sensors comprises connections to the data bus (7) through conductive yarns in "zig-zag".

In a preferred embodiment one or more of the referred sensors comprise connections to the data bus (7) through a welding on the sensor tail.

In a preferred embodiment one or more of the referred sensors comprises porous epoxy encapsulation.

In a preferred embodiment one or more of the referred sensors comprises protection by silicone adhesive.

In a preferred embodiment one or more of the referred sensors comprises a base of flexible PCB.

A preferred embodiment comprises sensors and data processing means for one or more of the following measures: body mass index, user's weight, the amount of calories consumed in total training time, training time, calories burned per second, instant travel speed, monitoring GPS tracker, travel distance, step count, record historical data, and / or calibration and measurement of body mass in motion.

### Background of the Invention

US2008287832A1 describes footwear (4) (or sole) that has multiple pressure sensors (8), which are connected to a device, "medical node" (6) that generates pressure data. The "medical node" transmits the pressure data to a base station (26). The base station analyzes the pressure data and can generate alerts, including text messaging, when the pressure data indicate that the pressure is beyond a certain level. The detection and pressure warning happens either at rest, eg. sitting, or in the exercise condition, for monitoring the condition of the patient, eg. diabetic or other medical problems. The invention allows that the excess pressure on a certain part of the foot of the patient can be avoided, and injuries to the skin and other adjacent tissues can be avoided too.

US5546955A describes a sock that includes a temperature sensor and a visual indicator connected to the sensor to provide an indication of the temperature of the leg. The sock also has a pressure application component that is automatically provided for periodic leg compression of the user. The invention allows the diagnosis of medical conditions, eg, phlebitis and prevents or reduces venous disease. Also inhibits the sock rolling down. It is not indicated any textile integration or how the operating problems in the specific environment of the foot / footwear are overcome.

FR2861846A1 describes a layer between foot and footwear, to obtain pressure signals exerted by one over the other, consisting of a layer of polyurethane gel (7) with pressure sensors (4) incorporated. The pressure sensors are solid and flat, positioned to detect the perpendicular pressure to the outer surface of the gel layer (not detecting cutting strains). It is foreseen the use in footwear and clothing (not explicitly socks), in boots or skiing or snowboarding boards, or even clothes for winter sports. The gel layer keeps the sensors close to the skin and adapts to user morphology. The gel layer is 2 - 10 mm thickness and hardness of 35-40 Shore-A. It is not indicated any textile integration or how the problems operating in the specific environment of the foot / footwear are overcome.

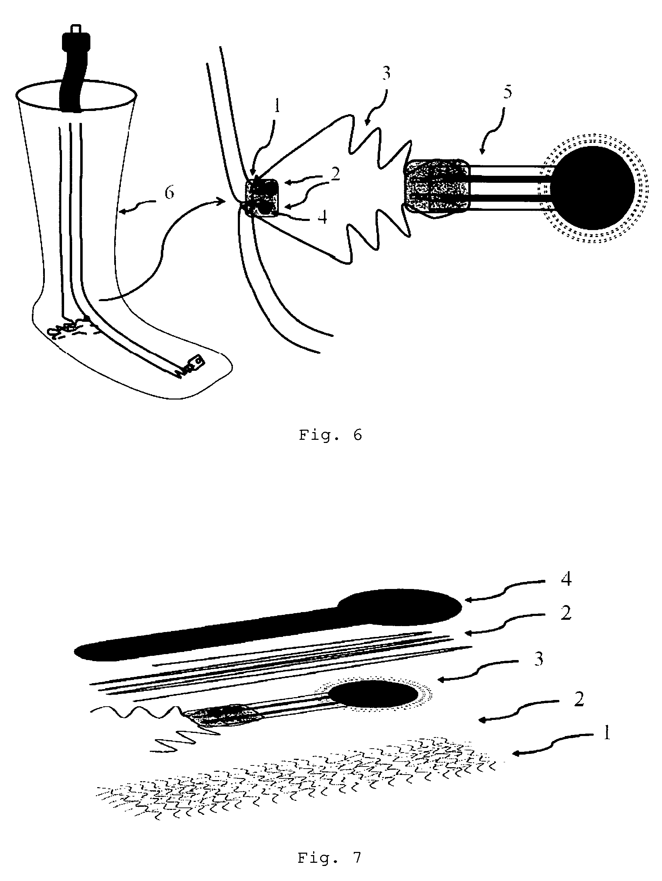

US6836744B1 describes a device (10), in which the movement of the back-foot and ankle, is handled by a processor and display (40) for the calculation of kinematic data that allow to identify particular movements of pronation and supination of the foot. The collection unit of plant pressure (30) allows you to calculate the plant pressure data to identify a center of the line pressure and even abnormal and excessive loads on the sole of the foot. Useful for measuring and analyzing human march, identifying the march style, in athletics and sports medicine rehabilitation, useful to measure body weight, and biomedical and podiatric uses, for orthopedic diagnostic and motor rehabilitation. It can be used with or without shoes. See figure 6 that may seem like a sock, but it can also be a kind of slipper. It is not indicated any textile integration or how the problems operating in the specific environment of the foot / footwear are overcome.

### General description of the invention

The present invention relates to a sock with integrated biometric monitoring.

The invention includes a biometric sensor system integrated into a sock with the ability to process and store the biometric data collected, in order to provide useful information to the user, relating the user's biometric data to performance parameters of physical effort.

The invention includes sensing integrated into the sock textile structure including temperature, heart beating and exerted pressure by the foot of the user, to be processed, interpreted and sent to a mobile device that does the data storage and allows the data visualization.

The invention includes textile integration of biometric monitoring devices through conductive fiber or yarns, woven and / or knitted in the sock and their own connections to sensors, terminals and encapsulations.

Despite the market already offering various solutions in shoes and insoles, these solutions have many limitations. The shoe does not fit the anatomy of the foot, which differs from person to person, we know that there are not two equal feet. On the other hand, for the reasons already mentioned, the foot never has the same position in the shoe. The foot slides inside the shoe. Another reason has to do with people's need to change with frequency of shoes that present different designs, materials, hardness, and mold qualities from manufacturer to manufacturer. Moreover, the shoe/insole are much more expensive than the sock, so less suitable for solutions involving greater rotation of the device.

For these reasons it is very difficult to position different sensors and thus compromising the efficient collection of physiological signals necessary for the project when using known solutions involving biometric monitoring shoes / insoles.

On the other hand, the sock has the following advantages:
- Due to sock's elasticity it fits to any foot anatomy.
- Can be used with or without shoes.
- Can be used in any shoe.
- A sock with sock characteristics usually designated as Eco-High-Tech, has properties that provide comfort, elasticity, resistance and therefore the ideal position to focus all the different sensors needed for the collection of physiological signals.
- It is a cheap solution, compared with the solution shoe / insole.

For these reasons the sock is a better solution for placing sensors and efficiently collecting the physiological signals of the foot.

However, the foot, and their footwear, constitute a technically difficult electronics operation environment. The humidity and temperatures in use are high, but can be markedly lower when not in use, the foot takes the equipment to high strains, including shear forces and impacts, and the own foot, while walking, significantly changes its shape, creating bends, sprains, strains.

The prescribed solution comprises the textile integration in the own sock of the electronic devices, including the conductors themselves that are preferably woven and / or the knitted in the sock.

The market offers several knitting technologies, but a solution was required that would allow vertical knitting of the conductive yarns to carry electricity and get data from different sensors employed, without any interruption of power supply and without compromising design and sock comfort.

The technological solutions presented by the market do not allow any solution that meets these requirements.

Although it would be possible to knit in "spiral" with four feeders, but the cost would be prohibitive, and the design and sock comfort would be compromised.

The solution was to adapt existing technology and allow the knitting of the electrical conductive yarns vertically so we can make the power supply to the different sensors and not compromising quality, comfort and design in the sock. Other solutions such as "add-on" could be equated, but it is known that the quality, design and comfort would be compromised, so the textile integration, namely the weave and / or knitting of the conductive yarns along the sock has specific advantages for this purpose.

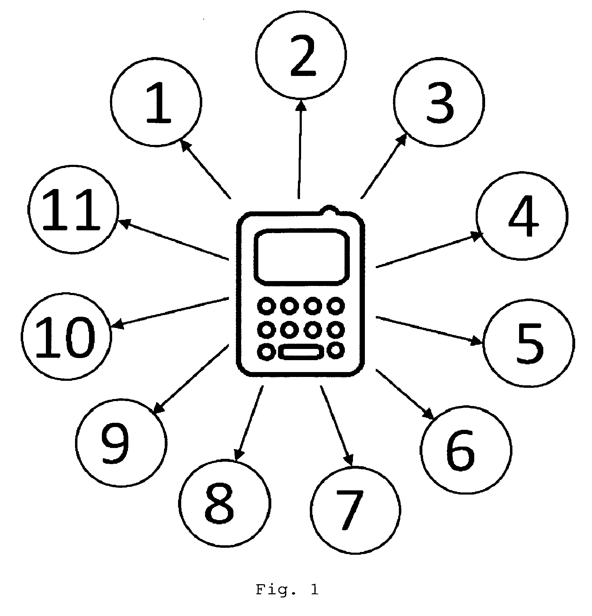

Through the developed software for this application it is possible to visualize all data and use them together with user input, to get the values of the user's weight, body mass index (BMI), calibration and measurement of body mass in motion, instantaneous speed at which the user moves, distance, step count, training time, calories burned per second and the amount of calories consumed in the total time of training (figure 1). The same software includes functions for *data logger,* and it's possible to export to a personal computer (PC) the data obtained through biometric sensors, being able to carry out an analysis of the data through the developed software for this purpose.

The tracker GPS (Global Positioning System) feature is also present in this system, if the mobile device (mobile phone) has an integrated GPS, the developed software for the application allows the acquisition of the cardinal points that indicate the path taken by the user. When exported to a PC it's possible to view the route on a virtual map (eg Google Earth).

This sensing system consists of a set of integrated sensors into the sock, an electronic central module that contains all the electronics needed to operate the device, which is placed on the ankle of the user, and a software to be installed on a mobile device (PDA, Smartphone, Ultra Mobile PC and Portable Console) or in bench devices (Desktops and Laptops), which will allow the visualization of all collected data and results of calculations made with them. The data transmission between the electronic central module to the mobile device is done through the communication protocol wireless data Bluetooth, using in specific cases 802.11 (WiFi) modules or ZigBee radio frequency (433MHz / 870MHz).

### Sensorization

They are collected preferably three distinct types of data using for this purpose preferably three specific sensors for each one of them.

### Temperature Sensor

The temperature values are preferably acquired through a commercial semiconductor developed for this purpose. This sensor converts the measured values of temperature into a digital signal easily interpretable by the processing practice, transmitted using the communication protocol "1-wire protocol." The range of temperature values measured by the sensor temperature is between -55 ° C and 125 ° C in a range of relative humidities from 20% to 95% being used resolution of 0.5 °C for ordinary users and 0.0625 ° C in applications for medicinal purposes.

In environments with high electromagnetic noise the current sensor is preferably replaced by others more immune to noise, using the communication protocol I ² C or analog sensors, with the additional insertion of one more conductive yarn or over a analog to digital conversion stage, respectively.

The temperature sensor is preferably integrated in a flexible PCB, which is then attached / welded to the conductive yarns incorporated into the structure of the sock, and making the connection of the sensor signal to the hardware. The whole structure of the flexible PCB with integrated sensor is preferably encapsulated in silicone neutral pH film (standard grade) through a process of lamination and / or molding process, which allows the waterproofing of the entire sensor- PCB flexible structure.

### Pressure Sensor

The pressure values exerted by the foot of the user are preferably collected by a piezoresistive sensor that generates a variation in the level of electrical resistance at its terminals, when it is applied pressure. The ranges of pressure sensors vary between 700 kPa of nominal pressure to 1300Kpa of maximum impact, however these values vary depending on the area of the used sensor, the larger the sensor, higher resolution will be, nevertheless having to withstand higher pressures.

To be able to interpret the values generated by this sensor, is preferably necessary to use a "Wheatstone bridge" connected to an instrumentation amplifier, which converts the sensor resistance variation in a voltage analog signal interpretable by an analog to digital converter (ADC) of the processing floor. As an alternative to the piezoresistive sensor it is possible to use a capacitive sensor, with a change, replacing the "Wheatstone Bridge" and amplification by a capacitance to digital converter that connects directly to the processing floor. The sensor preferably has a thickness less than 1.25mm in order to optimize the integration of the sock structure and allowing greater user comfort. The sensor is preferably integrated in the heel area and / or area of the metatarsal of foot in order to maximize the signal / response of the piezoresistive / capacitive sensor when the mechanical requests such as walking. The sensor is preferably encapsulated in silicone in silicon neutral pH film (standard grade) through a process of lamination and / or molding process, which allows the waterproofing of all sensors. The terminal / electrode sensors are preferably welded / connected to the integrated conductive yarns in the sock structure, carrying out the sensor driving signal to the hardware.

### Heart Beat Sensor

The heart beat sensor is preferably embedded in a rigid PCB, and preferably located at the ankle zone in contact with skin. This PCB is preferably encapsulated with silicone, so we can eliminate a possible discomfort caused to the user as this is in direct contact with skin. The PCB can be flexible, requiring special care with regard to the points of welding of the constituent components of the electronic sensor, which should preferably be strengthened. This module is preferably made up of the sensing floor with technology based on optical principle, commonly known as photoplethysmography (PPG), and in this case based on the method by reflectance. For implementing PPG method are preferably used two light emitting diodes (Light Emitting Diodes - LEDs) and a receiver / converter of light into an electrical signal (signal amplitude) of TAOS. This process consists of the light emitted by the two LEDs projected against the skin, where part of this light is absorbed by the skin, the rest being reflected and detected by the receiver. The variation that exists in the amplitude of the signal (in the receiver output), which corresponds to a greater or lesser absorption of light, will dictate whether or not there was a cardiac pulse.

This module is preferably constituted by three floors, the sensing floor (referred above), the signal conditioning floor and finally the processing / communication floor.

The sensing floor contains the light emitters and receiver and is responsible for the entire process of converting light into an electrical signal. The conditioning floor is responsible for all signal conditioning stages, since filtering, amplification and signal conversion (analog to digital conversion - ADC), which is necessary to obtain interpretable signal by the processing/communication unit. The processing / communication floor has two main functions, the interpretation of the digital signal for the heart beat over time in order to extract the relevant information, and the encapsulation of information extracted according to the asynchronous serial communication protocol, so that the processed data can be sent correctly to the central electronic module. This data is sent in digital format, and is no need any signal conditioning floor for your reception.

### Central electronic module

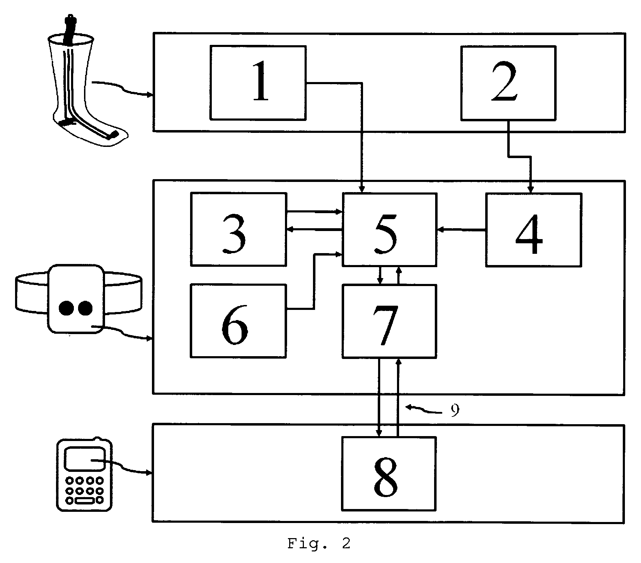

The central electronic module does the acquisition of the signals from the sensors, their conditioning /processing, the data processing and finally the optional communication to a mobile device. In this module there is also preferably one monitorization floor of the charge level of your battery power, and this information is also sent to the mobile device (figure 2). The central electronic module is preferably encapsulated in a solid polymer structure (box) which is preferably fixed at the ankle zone using an elastic band fixation by pins or an elastic band with adjustable Velcro fastening, or an elastic band adjustable clamping polymer springs. The hardware module is thus preferably removable, but may not be washable, unlike the rest of the sock structure is preferably washable.

The data of the temperature and pressure sensors integrated in the sock structure are conducted / transmitted preferably to the hardware through conductive textile yarn integrated into the yarn sock structure. The sensor signal is conducted through the conductive textile yarn in the sock structure that is preferably attached / welded to one end of flexible elastic band or a conductive encapsulated yarn. The conductive elastic band preferably has, at the other end, a rigid connector pins and / or a mini-USB connector, and in the case of the conductive encapsulated yarn this will have preferably a mini-jack connector on the other end, which are systems that connect to the hardware module. This system allows a greater comfort for the user in establishing the physical connection between the conductive yarns in the sock structure, which transmit signals from the sensors in the sock structure, and the hardware module. Thus, it becomes easy for the user to decouple the textile sock part that is washable from the module hardware non washable, through the referred connectors.

### Power supply and battery charge monitoring

Batteries integrated into the hardware module are responsible for feeding all the electronic and sensing systems in the sock. They are preferably used lithium-ion batteries or lithium - polymer with volumes greater than 8 cm³ and less than 50 cm³, and load values between 100mAh and 500mAh depending on the desired autonomy.

This floor has the functionality to monitor the charge level of battery power of the central electronics module sending its status to the mobile device. For this purpose it is used a device that monitors the charge level and sends signals to the processing floor if the battery falls below a set value, or if the battery is charging, sends a battery load signal and load complete signal. On this floor is also included in the control circuit power supply module, which is able to detect the presence of a charging unit and make the battery charging power.

### Analog signal conditioning

Of the three sensors included in the system preferably only the pressure sensor requires signal conditioning in this module. This conditioning signal is preferably done using a "Wheatstone bridge" connected to an instrumentation amplifier that permits to detect the resistance the variation of the pressure sensor when pressure is requested on it, having in the amplifier output an analog signal interpretable by analog to digital converter present in the microcontroller. This analog signal generated is characterized by reading a voltage of 0 V when there is no pressure and supply voltage when the system reached the maximum pressure value.

### Processing and treatment of the sensor signals

To perform all processing signals generated by the system, both sensors of the power supply floor of battery power monitoring floor, it is employed preferably a microcontroller that due to the modules included, allows the proper interpretation of signals.

The processing is specifically made for each of the different signals. The signal from the temperature sensor will be preferably received in a digital port of the microcontroller, which contains the necessary algorithm to receive correctly the sent values and convert them into appropriate values for later transmission to the mobile device.

The heartbeat signal is already pre-treated by the module itself, and is only required to receive the values in a digital port and forward them to the mobile device.

The pressure sensor signal requires the use of the analog to digital converter present in the microcontroller, having implemented a detection algorithm of the signal transition referent to the act of exercising and removing pressure. From the detection of these transitions, it was possible to develop algorithms for the detection of steps and timing of each step and processing of such data to send relevant and interpretable information for use by mobile device software. Through a continuous analysis algorithm of pressure in the high-level transitions is made a rough calculation of body mass preferably having a basis of comparison one initial calibration to the test object individual.

The signals of low battery, battery charging and battery charged, are also preferably acquired directly by the microcontroller digital ports, which has also developed an algorithm for interpretation of these data and preparation for sending to the mobile device.

### Data communication to the mobile phone by Bluetooth

In order to be able to communicate between the central electronic module and the mobile device, a device for data transmission is used preferably by Bluetooth in the former, which communicates preferably with the microcontroller by an asynchronous serial protocol, receiving a frame of data properly structured to be interpreted by the software of the mobile device. In some specific cases, 802.11 (WiFi) modules, ZigBee or radio frequency (433MHz / 870MHz) are preferably used, allowing direct connection to a specific device or a PC that does not have the communication protocol Bluetooth, but Wi-Fi.

### Postprocessing software and data visualization obtained in the Sock

To be able to visualize by the user the generated and processed data in hardware modules, was developed a software for mobile devices with a user-friendly graphical interface. This software is preferably optimized for the Windows Mobile platform, but can also be adapted to other platforms, including Android, Symbian OS and iPhone OS.

The connection / communication software-sock is preferably established automatically, following a concept of "Plug and Play", requiring only a prior pairing with the central electronic module.

The software is preferably based on windows concept, thus each one of the features is grouped for windows as follows: setup windows and monitoring windows. Specifying, the configuration is related to user data and communication; the monitoring is related to presentation of all data relevant to the user, obtained in the sock. The user-interaction software is preferably done through the navigation buttons on the mobile device, but it can also be done via a touch screen if the device has that kind of physical interface. This quite intuitive, the user can easily navigate through all available windows.

The data processing requires more processing time, including the development of mathematical calculations used to obtain values such as BMI, level of calories burned, training time or, are made preferably by the mobile device software, which has an higher processing speed than the microcontroller present in central electronic module.

Calculations and data supplied to the user by the software interface are obtained from the sensors, calculated from sensor data or calculated from sensor data together with data entered by the user.

### Component integration in the Sock

To ensure that the entire textile structure of the sock is washable according to current international standards, it is necessary to proceed to the waterproofing and encapsulation of sensors and electrical driving signal systems in the sock structure.

The temperature sensor is preferably welded to a flexible PCB which is then connected to the conductive textile yarn incorporated into the sock structure. The temperature sensor is then preferably encapsulated in silicone neutral pH film (standard grade) through processes of lamination, hot and / or normal temperature pressing, or molding, which allows the waterproofing of the sensor, the PCB and joints with conductive textile yarns. Later, this system is preferably encapsulated between the textile structure of the sock and a textile structure of support, constructed and constituted by the same sock material, through a process of lamination and / or sealing and finishing the process of encapsulation in the textile structure.

The contacts / electrodes of the pressure sensors are preferably connected to a flexible PCB which in turn connects to the conductive textile integrated into the sock structure, being the sensor, the PCB and the connection sensor-conductive textile yarn preferably encapsulated in silicone PH neutral film (standard grade) through processes of lamination, hot and / or normal temperature pressing, or molding, which allows the waterproofing of sensor and joints with textile conductive yarns. Subsequently this system is preferably encapsulated between the textile structure of the sock and a textile structure of support, constructed and constituted by the same sock material, through a process of lamination and / or sealing and finishing the process of encapsulation in the textile structure.

It was selected a conductive textile knittable yarn by conventional knitting processes, such as the conductive yarn to use to drive the sensors signal to the elastic band and / or conductive encapsulated yarn, which later lead the sensors signal integrated into the sock to the hardware / module control. The selected yarn is preferably encapsulated and waterproof, allowing the sock to be washable. The yarn is preferably integrated into the sock structure through conventional knitting processes and following a pattern of preferably three or more courses of conduction signals between the sensors and conductive bands / encapsulated signal conductive yarns to the hardware. This yarn is preferably welded to the contacts of a PCB and then to the contacts of pressure sensors, and welded to the PCB contacts of the temperature sensor, and being subsequently welded to the contacts of the conductive band / encapsulated yarn.

The conductive knittable yarns are preferably connected to an elastic band that has the same number of conductive yarns in the knitted sock. The purpose of this band is to drive the electrical signal from the sensors to the central module. Being one of the ends preferably welded and waterproofed to the knitted yarn in the sock through the mentioned processes above, the other end is plugged to a connector that will do the interface between the elastic band and the electronic central module. The standard connector used is preferably the MINI or MICRO-USB being mostly used in consumer electronics devices, allowing to easily charge the electronic device through a USB port on a PC.

Other jack, pin, or "Plug and Play" connectors may be used depending on the type of elastic used band, or the replacement of the same, or a conductive cable.

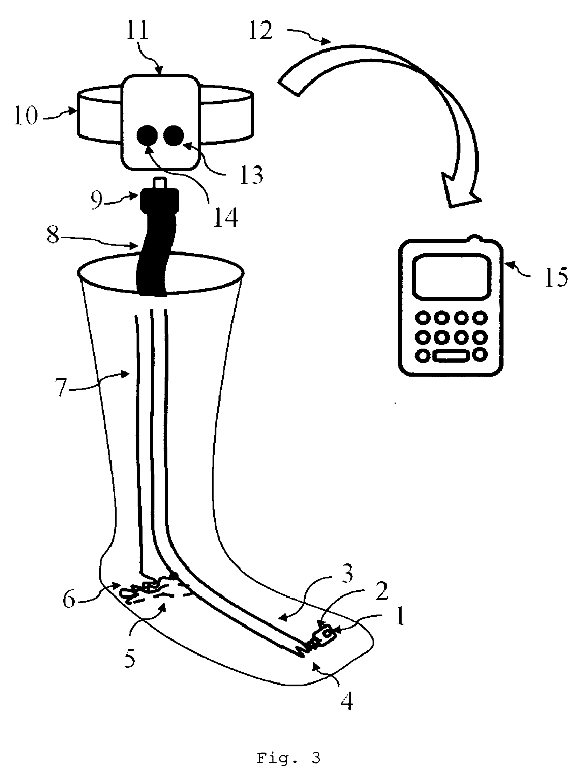

All electronics are preferably encapsulated in a box being necessary its removal before washing the sock, if the box is not waterproof / washable. The box is preferably secure to an elastic band, which is secured to the ankle of the user, so it is preferred that the elastic band hugs the ankle and is attached by Velcro. This solution allows for easy removal of the electronic and avoids forgetting to remove it before washing, being an alternative the direct fixing of the electronic module to the sock through click connectors as visible in figure 3.

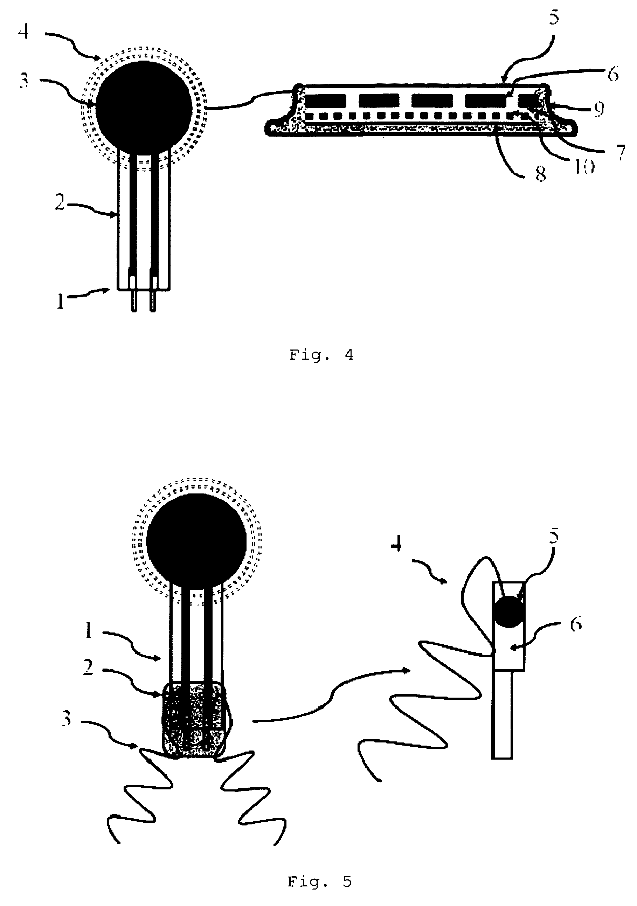

### Encapsulation of the pressure sensor (Fig. 4)

- The pressure sensor is based in FSR technology (Force Sensitive Resistor) (3);
- The sensor is composed of different layers: protective layer (5), active layer (6), spacer (7), printed electrodes (10) and plastic substrate (8);
- The spacer allows the resistant / resilient work of the pressure sensor (3), having an air vent at the end;
- It is preferred a polymer encapsulation (9) on the sensor, to have durable, washable and shear stress resistant devices (cutting forces).

### Problems:

- Even if the sensor has a spacer (7) with an air vent in the extremity, the encapsulation process of the sensor blocks the air vent;
- If the air vent is blocked, the system is washable;
- But, if the vent is blocked, the sensor (3) will not behave correctly when the pressures are applied consecutively on him, because a vacuum is caused and the empty space between the active area (6) and the printed electrodes (10) disappears;
- It is necessary a washable encapsulated sensor that responds to consecutive pressure correctly and avoid creating a vacuum between the layers of active area (6) and printed electrodes (10).

### Solution:

- The encapsulation process was done with a mold, to apply an epoxy resin (preferably 3M Scotch-Weld DP-190) (9) on the border with the sensor (4);
- The curing process is accomplished by oven at 60 ° C for about two hours;
- The epoxy resin (4) reaches an acceptable porosity after curing;
- The porosity allows that the air enters inside the sensor, and prevents the ingress of water.

### Connection "Sensor - conductive yarn" (Fig. 5)

### Features:

- The end of the flexible sensor allows the with connection conductive yarns, preferably by welding.

### Problems:

- The conductive yarn (3) used for welding to the electrodes sensor is highly flexible;
- The conductive yarn (3) should allow welding;
- The weld area (5), after the welding process, becomes rigid and therefore brittle;
- The tensile forces in the final product (the sock is based on a stretchy and elastic textile) are the main problems for the stability of the welded region;
- The conductive yarn (3) should be washable;
- The conductive yarn (3) should be knittable.

### Solution:

- After extensive testing of various yarns, it was concluded that the best conductive yarn (3) for this application, which corresponds to the desired characteristics is preferably NOVONIC and has the following preferred characteristics: Cu / Ag 0.04 mm and textile polyester dtex 50/24/1 - 3 ohm / meter;
- The improvement of the welding process was made, changing the path of the conductive yarn (3) through the tail of the sensor (1);
- The conductive yarn (3) comes into contact laterally with the tail of the sensor (1) and travels through the tail of the sensor until the terminals (4);
- The welding is done only at the tail end of the sensor, where the conductive yarn is connected to sensor terminals;
- The welded area (5) is then preferably encapsulated with an epoxy resin (preferably 3M Scotch- Weld DP-190) (2);
- The welded area (5) is thus protected from disruptive and traction forces, and the conductive yarns (3) maintain their flexibility to be embedded into the textile substrate;
- The connection between the conductive yarn and the sensor thus becomes robust.

### Connection "conductive yarn - bus" (Fig. 6)

### Features:

- The connection between the conductive yarn and the data bus is made by welding;
- The data bus is preferably composed of 3 or 4 yarns, and is based in the already mentioned conductive yarn for connection to the sensor;
- The conductive yarn is embedded in the fabric, preferably woven or knitted;
- The yarn is preferably NOVONIC and has the following features: Cu / Ag 0.04 mm and textile polyester dtex 50/24/1 - 3 ohm / meter.

### Problems:

- The yarn is highly flexible and the weld area, after the welding process, becomes rigid and therefore brittle;
- The connection between flexible and rigid components, usually results in feeble and instable couplings;
- As the conductive yarns are embedded in the fabric, these must be not only flexible but also elastic / extensible. Solution:
- The conductive yarn from the sensor (5) goes through the textile sector (6) in zigzag shape (3), to allow flexibility when the mesh is stretched;
- When the yarn reaches the data bus, a loop / ring (1) is made;
- The yarn data bus is also drawn to create a loop (1);
- The welding is done at the top part of the yarn data bus (2) loop;
- An encapsulation with epoxy resin is made over the welded area (4).

### Protection of a sensor (Fig. 7)

### Features:

- The sensor (3) can be of various types and for various measurements, and is incorporated into a fabric substrate (1).

### Problems:

- As the sensor (3) is from plastic and slightly three-dimensional, ie has a non-negligible thickness, the fabric integration (1) results in two very different surfaces, including its thickness;
- One or more of the major applications of the present invention involves using fabric near the skin;
- The plastic surface of the sensor cannot be comfortable for the user;
- The boundaries of the sensor can cause skin lesions, due first to the three-dimensional shape and because a straight and regular contact may be occurring;
- The sensor, as a whole, is not resistant and washable. Solution:
- To protect from water, both surface and bottom of the sensor, a silicone adhesive (2) waterproof and breathable is applied;
- The silicone adhesive (2) at the bottom allows a lamination process of the sensor (3) in the fabric (1);
- The silicone adhesive (2) at the top allows a lamination process of the sensor in a protective mesh (4);
- The mesh (4) is preferably in *Jersey,* with a base preferably in polyamide (98%) and spandex (2%);
- The silicone adhesive (2) protects all sensor (3) from water and its welding areas, and allows air to enter, to avoid the vacuum effect in the inner layer of the sensor.

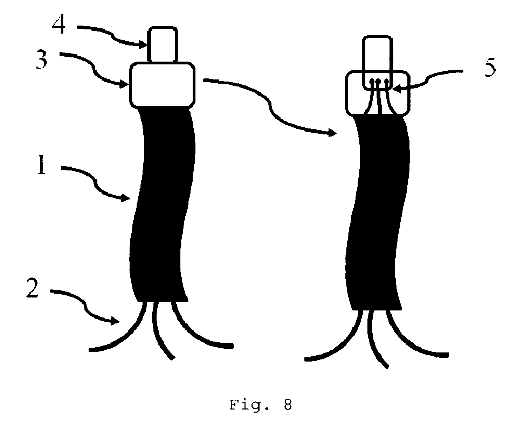

### Conductive connection band (Fig. 8)

### Features:

- The conductive connection band (1) connects the conductive yarns (2) of the data bus of the sock and the connector, preferably USB (mini or micro) (4), as a way to allow travel of the conductive yarns (2).

### Problems:

- The data bus, consisting of several yarns (2), needs to connect a USB connector (4);
- The connection between the flexible yarns (2) and a rigid module, as a USB connector (4), must be robust and esthetic.

### Solution:

- The conductive yarns (2) are flexible to allow their protection to mechanical forces, are incorporated into a conductive connection textile band (1);
- The textile band (1) has "tunnels" through which the conductive yarns (2) can pass;
- The textile band becomes a conductive band (1) with the integration of conductive yarns (2) through it;
- The connector, preferably USB (4), is fixed by a welding process (5) on the conductive connection band (1) to enable connectivity;
- After the connection (5), the injection molding is done to create a robust and esthetic encapsulation (3), and the connection is thus protected.

### Description of the Figures

For an easier understanding of the invention there are attached together the figures, which represent preferred embodiments of the invention which, however, are not intended to limit the scope of this invention.
**Figure 1****:** Schematic representation of the collected data and where
   (1) represents the body mass index,
   (2) represents the user's weight,
   (3) represents the amount of calories consumed in the total training time,
   (4) represents the training time,
   (5) represents the calories consumed per second,
   (6) represents current speed of travel,
   (7) represents the follow-up GPS tracker,
   (8) represents the distance traveled,
   (9) represents step count,
   (10) represents the historical data record, and
   (11) represents calibration and measurement of body mass in motion.
**Figure 2****:** Schematic representation of the main modules of the monitoring system where
   (1) represents the temperature sensor,
   (2) represents the pressure sensor,
   (3) represents the heartbeat sensor,
   (4) represents analog signal conditioning,
   (5) represents processing and data treatment,
   (6) represents power and load monitoring,
   (7) represents the interface for wireless data communication, eg. mobile by bluetooth,
   (8) represents post-processing and data visualization,
   (9) represents a wireless data communication, eg. bluetooth.
**Figure 3****:** Schematic representation of the invention where
   (1) represents a temperature sensor,
   (2) represents the PCB support board,
   (3) represents conductive yarns,
   (4) represent conductive yarns in zig-zag
   (5) represents a pressure sensor,
   (6) represents conductive yarns,
   (7) represents a data bus,
   (8) represents a conductive connection band,
   (9) represents a connector, eg. USB
   (10) represents an elastic band,
   (11) represents a box for electronics,
   (12) represents a wireless connection,
   (13) represents a led,
   (14) represents a button, eg. on / off, and
   (15) represents a mobile device, eg. *Smartphone.*
**Figure 4****:** Schematic representation of encapsulation and connection of the pressure sensor where
   (1) represents sensor terminals,
   (2) represents the tail of the sensor,
   (3) represents the pressure sensor,
   (4) represents epoxy resin,
   (5) represents protective material,
   (6) represents the active area of sensor,
   (7) represents a spacer,
   (8) represents the plastic substrate,
   (9) represents epoxy resin, and
   (10) represents electrodes.
**Figure 5****:** Schematic representation of the connection between the conductive band and a sensor where
   (1) represents the tail of the sensor,
   (2) represents epoxy resin,
   (3) represents conductive yarn,
   (4) represents the terminal detail,
   (5) represents the welding, and
   (6) represents the sensor terminal.
**Figure 6****:** Schematic representation of connection between the conductive bus and the conductive yarns where
   (1) represents loops or rings of conductive yarn,
   (2) represents the welding,
   (3) represents conductive yarn in zig-zag
   (4) represents epoxy resin,
   (5) represents a pressure sensor, and
   (6) represents the textile.
**Figure 7****:** Schematic representation of the sensor protection where
   (1) represents the textile,
   (2) represents a silicone adhesive,
   (3) represents a sensor, and
   (4) represents the knitting.
**Figure 8****:** Schematic representation of the conductive band where
   (1) represents the conductive connection band,
   (2) represents conductive yarns,
   (3) represents the encapsulation,
   (4) represents the connector, eg. USB
   (5) represents the welding.

### Encapsulation of the pressure sensor (Fig. 4)

- The pressure sensor is based in FSR technology (Force Sensitive Resistor) (3);
- The sensor is composed of different layers: protective layer (5), active layer (6), spacer (7), printed electrodes (10) and plastic substrate (8);
- The spacer allows the resistant / resilient work of the pressure sensor (3), having an air vent at the end;
- It is preferred a polymer encapsulation (9) on the sensor, to have durable, washable and shear stress resistant devices (cutting forces).

### Problems:

- Even if the sensor has a spacer (7) with an air vent in the extremity, the encapsulation process of the sensor blocks the air vent;
- If the air vent is blocked, the system is washable;
- But, if the vent is blocked, the sensor (3) will not behave correctly when the pressures are applied consecutively on him, because a vacuum is caused and the empty space between the active area (6) and the printed electrodes (10) disappears;
- It is necessary a washable encapsulated sensor that responds to consecutive pressure correctly and avoid creating a vacuum between the layers of active area (6) and printed electrodes (10).

### Solution:

- The encapsulation process was done with a mold, to apply an epoxy resin (preferably 3M Scotch-Weld DP-190) (9) on the border with the sensor (4);
- The curing process is accomplished by oven at 60 ° C for about two hours;
- The epoxy resin (4) reaches an acceptable porosity after curing;
- The porosity allows that the air enters inside the sensor, and prevents the ingress of water.

### Connection "Sensor - conductive yarn" (Fig. 5)

### Features:

- The end of the flexible sensor allows the with connection conductive yarns, preferably by welding.

### Problems:

- The conductive yarn (3) used for welding to the electrodes sensor is highly flexible;
- The conductive yarn (3) should allow welding;
- The weld area (5), after the welding process, becomes rigid and therefore brittle;
- The tensile forces in the final product (the sock is based on a stretchy and elastic textile) are the main problems for the stability of the welded region;
- The conductive yarn (3) should be washable;
- The conductive yarn (3) should be knittable.

### Solution:

- After extensive testing of various yarns, it was concluded that the best conductive yarn (3) for this application, which corresponds to the desired characteristics is preferably NOVONIC and has the following preferred characteristics: Cu / Ag 0.04 mm and textile polyester dtex 50/24/1 - 3 ohm / meter;
- The improvement of the welding process was made, changing the path of the conductive yarn (3) through the tail of the sensor (1);
- The conductive yarn (3) comes into contact laterally with the tail of the sensor (1) and travels through the tail of the sensor until the terminals (4);
- The welding is done only at the tail end of the sensor, where the conductive yarn is connected to sensor terminals;
- The welded area (5) is then preferably encapsulated with an epoxy resin (preferably 3M Scotch- Weld DP-190) (2);
- The welded area (5) is thus protected from disruptive and traction forces, and the conductive yarns (3) maintain their flexibility to be embedded into the textile substrate;
- The connection between the conductive yarn and the sensor thus becomes robust.

### Connection "conductive yarn - bus" (Fig. 6)

### Features:

- The connection between the conductive yarn and the data bus is made by welding;
- The data bus is preferably composed of 3 or 4 yarns, and is based in the already mentioned conductive yarn for connection to the sensor;
- The conductive yarn is embedded in the fabric, preferably woven or knitted;
- The yarn is preferably NOVONIC and has the following features: Cu / Ag 0.04 mm and textile polyester dtex 50/24/1 - 3 ohm / meter.

### Problems:

- The yarn is highly flexible and the weld area, after the welding process, becomes rigid and therefore brittle;
- The connection between flexible and rigid components, usually results in feeble and instable couplings;
- As the conductive yarns are embedded in the fabric, these must be not only flexible but also elastic / extensible. Solution:
- The conductive yarn from the sensor (5) goes through the textile sector (6) in zigzag shape (3), to allow flexibility when the mesh is stretched;
- When the yarn reaches the data bus, a loop / ring (1) is made;
- The yarn data bus is also drawn to create a loop (1);
- The welding is done at the top part of the yarn data bus (2) loop;
- An encapsulation with epoxy resin is made over the welded area (4).

### Protection of a sensor (Fig. 7)

### Features:

- The sensor (3) can be of various types and for various measurements, and is incorporated into a fabric substrate (1).

### Problems:

- As the sensor (3) is from plastic and slightly three-dimensional, ie has a non-negligible thickness, the fabric integration (1) results in two very different surfaces, including its thickness;
- One or more of the major applications of the present invention involves using fabric near the skin;
- The plastic surface of the sensor cannot be comfortable for the user;
- The boundaries of the sensor can cause skin lesions, due first to the three-dimensional shape and because a straight and regular contact may be occurring;
- The sensor, as a whole, is not resistant and washable. Solution:
- To protect from water, both surface and bottom of the sensor, a silicone adhesive (2) waterproof and breathable is applied;
- The silicone adhesive (2) at the bottom allows a lamination process of the sensor (3) in the fabric (1);
- The silicone adhesive (2) at the top allows a lamination process of the sensor in a protective mesh (4);
- The mesh (4) is preferably in *Jersey,* with a base preferably in polyamide (98%) and spandex (2%);
- The silicone adhesive (2) protects all sensor (3) from water and its welding areas, and allows air to enter, to avoid the vacuum effect in the inner layer of the sensor.

### Conductive connection band (Fig. 8)

### Features:

- The conductive connection band (1) connects the conductive yarns (2) of the data bus of the sock and the connector, preferably USB (mini or micro) (4), as a way to allow travel of the conductive yarns (2).

### Problems:

- The data bus, consisting of several yarns (2), needs to connect a USB connector (4);
- The connection between the flexible yarns (2) and a rigid module, as a USB connector (4), must be robust and esthetic.

### Solution:

- The conductive yarns (2) are flexible to allow their protection to mechanical forces, are incorporated into a conductive connection textile band (1);
- The textile band (1) has "tunnels" through which the conductive yarns (2) can pass;
- The textile band becomes a conductive band (1) with the integration of conductive yarns (2) through it;
- The connector, preferably USB (4), is fixed by a welding process (5) on the conductive connection band (1) to enable connectivity;
- After the connection (5), the injection molding is done to create a robust and esthetic encapsulation (3), and the connection is thus protected.

## Claims

1. Sock for integrated biometric monitoring comprising:
- sock with a data bus (7) which comprises one or more conductive yarns (3) knitted within the sock;
- one or more biometric sensors (1, 5);
**characterized in that** the conductive yarns are encapsulated and waterproof yarns knitted vertically along the sock and each sensor comprises porous epoxy encapsulation and protection by silicon adhesive, wherein the silicon adhesive is waterproof and breathable.

2. Sock according to claim 1, wherein the sock further comprises a data processing device (11), a conductive connection band (8) through which a connection is made to the data bus (7), and a wireless interface (12) to a mobile device (15).

3. Sock according to the previous claim, wherein the data processing device (11) comprises one or more visual LED indicators (13) and one or more buttons (14), wherein the one or more buttons comprise an on / off switch.

4. Sock according to any previous claim, wherein the one or more biometric sensors comprise a pressure sensor (5) and a temperature sensor (1).

5. Sock according to any previous claim, wherein the one or more biometric sensors comprise connections to the data bus (7) through conductive yarns in zig-zag.

6. Sock according to any previous claim, wherein the one or more biometric sensors comprise connections to the data bus (7) through a welding on the tail of the sensor.

7. Sock according to any previous claim, wherein the one or more biometric sensors comprise a flexible, printed-circuit-board base.

8. Sock according to any claim 2, wherein the one or more biometric sensors and the data processing device are configured for one or more of the following: to measure body mass index, user's weight, the amount of calories consumed in total training time, training time, calories burned per second, or instant travel speed; to monitor a GPS tracker, travel distance, or step count; to record historical data or calibration and measurement of body mass in motion.

## Patentansprüche

1. Socke für die integrierte biometrische Überwachung umfassend:
- Socke mit einem Datenbus (7), die ein oder mehrere leitfähige innerhalb der Socke gewirkten Garne (3), umfasst;
- ein oder mehrere biometrische Sensoren (1, 5); **dadurch gekennzeichnet, dass** die leitfähigen Garne verkapselte und wasserfeste vertical entlang der Socke gewirkte Garne sind und dass jeder Sensor die poröse epoxy Verkapselung und den Schutz durch Silikonklebstoff umfasst, wobei der Silikonklebstoff wasserfest und atmungsaktiv ist.

2. Socke nach Anspruch 1, wobei die Socke ausserdem eine Datenverarbeitungsvorrichtung (11), ein leitfähiges Verbindungsband (8), mittels dem eine Verbindung mit dem Datenbus (7) hergestellt wird, und eine drahtlose Schnittstelle (12) zu einer mobilen Vorrichtung (15), umfasst.

3. Socke nach dem vorhergehenden Anspruch, wobei die Datenverarbeitungsvorrichtung (11) eine oder mehrere visuelle LED-Anzeigen (13) und eine oder mehrere Tasten (14) umfasst, wobei die eine oder die mehreren Tasten ein Ein-/Ausschalter umfassen.

4. Socke nach einem der vorhergehenden Ansprüche, wobei der eine oder die mehreren biometrischen Sensoren ein Drucksensor (5) und ein Temperatursensor (1) umfassen.

5. Socke nach einem der vorhergehenden Ansprüche, wobei der eine oder die mehreren biometrischen Sensoren Verbindungen zu dem Datenbus (7) mittels leitfähiger Garne in Zick-Zack umfassen.

6. Socke nach einem der vorhergehenden Ansprüche, wobei der eine oder die mehreren biometrischen Sensoren Verbindungen zu dem Datenbus (7) mittels einer Schweißung am Sensorende umfassen.

7. Socke nach einem der vorhergehenden Ansprüche, wobei der eine oder die mehreren biometrischen Sensoren eine flexible Leiterplattenbasis umfassen.

8. Socke nach einem der Ansprüche 2-7, wobei der eine oder die mehreren biometrischen Sensoren und die Datenverarbeitungsvorrichtung für eine oder mehrere der folgenden Maßnahmen konfiguriert sind: zur Messung des Body-Mass-Index, des Benutzergewichtes, der Menge an in der Gesamttrainingszeit verbrauchten Kalorien, der Trainingszeit, der pro Sekunde verbrannten Kalorien oder der augenblicklichen Reisegeschwindigkeit; zur Überwachung eines GPS-Trackers, der Reisedistanz oder der Schrittzahl; zur Registrierung historischer Daten oder Kalibrierung und Messung der Körpermasse in Bewegung.

## Revendications

1. Chaussette pour la surveillance biométrique intégrée comprenant:
- chaussette avec un bus de données (7) qui comprend un ou plusieurs fils conducteurs (3) tricotés à l'intérieur de la chaussette;
- un ou plusieurs capteurs biométriques (1, 5); **caractérisée en ce que** les fils conducteurs sont des fils encapsulés et résistants à l'eau tricotés verticalement le long de la chaussette et **en ce que** chacun des capteurs comprend l'encapsulation époxy poreuse et la protection par un adhésif de silicone, dans laquelle l'adhésif de silicone est résistant à l'eau et respirable.

2. Chaussette selon la revendication 1, dans laquelle la chaussette comprend en outre un dispositif de traitement de données (11), une bande de connexion conductrice (8), par l'intermédiaire de laquelle est établie une connexion avec le bus de données (7), et une interface sans fil (12) vers un dispositif mobile (15).

3. Chaussette selon la revendication précédente, dans laquelle le dispositif de traitement de données (11) comprend un ou plusieurs indicateurs LED visuels (13) et un ou plusieurs boutons (14), dans laquelle l'un ou les plusieurs boutons comprennent un interrupteur marche/arrêt.

4. Chaussette selon l'une quelconque des revendications précédentes, dans laquelle l'un ou les plusieurs capteurs biométriques comprennent un capteur de pression (5) et un capteur de température (1).

5. Chaussette selon l'une quelconque des revendications précédentes, dans laquelle l'un ou les plusieurs capteurs biométriques comprennent des connexions au bus de données (7) par l'intermédiaire de fils conducteurs en zig-zag.

6. Chaussette selon l'une quelconque des revendications précédentes, dans laquelle l'un ou les plusieurs capteurs biométriques comprennent des connexions au bus de données (7) par l'intermédiaire d'un soudage sur la queue du capteur.

7. Chaussette selon l'une quelconque des revendications précédentes, dans laquelle l'un ou les plusieurs capteurs biométriques comprennent une base de circuit imprimé flexible.

8. Chaussette selon l'une quelconque des revendications 2-7, dans laquelle l'un ou les plusieurs capteurs biométriques et le dispositif de traitement de données sont configurés pour une ou plusieurs des mesures suivantes: pour mesurer l'indice de masse corporelle, le poids de l'utilisateur, la quantité de calories consommées pendant le temps total d'entraînement, le temps d'entraînement, les calories brûlées par seconde ou la vitesse de parcours instantanée; pour surveiller un localisateur GPS, la distance de parcours ou le nombre de pas; pour enregistrer des données historiques ou l'étalonnage et la mesure de la masse corporelle en mouvement.
